(19) Europäisches Patentamt
European Patent Office
Office européen des brevets

(11) Publication number: 0 525 877 A1

(12) # EUROPEAN PATENT APPLICATION

(21) Application number: 92202225.6

(22) Date of filing: 20.07.92

(51) Int. Cl.5: C07D 317/46, C07D 317/66, C07D 317/72, C07D 319/08, C07D 327/04, C07D 327/06, A01N 43/30, A01N 43/32, C07D 317/62

(30) Priority: 23.07.91 GB 9115837

(43) Date of publication of application: 03.02.93 Bulletin 93/05

(84) Designated Contracting States: AT BE CH DE DK ES FR GB IT LI NL SE

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Anderson, Martin**
**92, Clare Road**
**Whitstable, Kent CT5 2EH(GB)**
Inventor: **Brinnand, Antony Grove**
**The Meads, Perry Wood, Selling**
**Faversham, Kent ME13 9RU(GB)**
Inventor: **WOODALL, Roger Ernest**
**1, Slips Cottage, Painters Forstal**
**Faversham, Kent ME13 OEW(GB)**

(54) **Pesticidal heterocyclic compounds.**

(57) Compounds of the general formula:

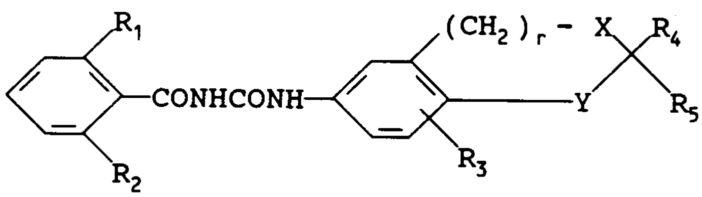

(I)

wherein $R_1$ represents a hydrogen or halogen atom; $R_2$ represents a halogen atom; $R_3$ represents a hydrogen or halogen atom or an alkyl group; $R_4$ and $R_5$ each independently represents a hydrogen atom or an alkyl, haloalkyl, alkoxy or aralkyl group; or $R_4$ and $R_5$ together form an alkylene group; X and Y each independently represents an oxygen or sulphur atom; and r is 0 or 1, with the proviso that at least one of $R_3$, $R_4$ and $R_5$ is not a hydrogen atom when both $R_1$ and $R_2$ represent a chlorine atom, X and Y both represent an oxygen atom and r is 0, have useful pesticidal activity.

EP 0 525 877 A1

The present invention relates to heterocyclic compounds having pesticidal, especially insecticidal and acaricidal, activity.

GB-A-1324293 claims compounds of the formula

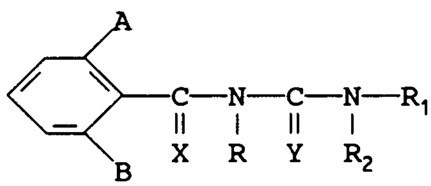

in which A and B may each represent a halogen atom, X and Y may each represent an oxygen atom, R may represent a hydrogen atom, $R_1$ may represent a hydrogen atom and $R_2$ may represent a phenyl group substituted with a dioxymethylene group, which are described as being biologically active and possessing insecticidal activity. However, in the only exemplified compound containing such a phenyl group substituted with a dioxymethylene group, A and B both represent chlorine atoms.

EP-A-0042533 claims compounds of the formula

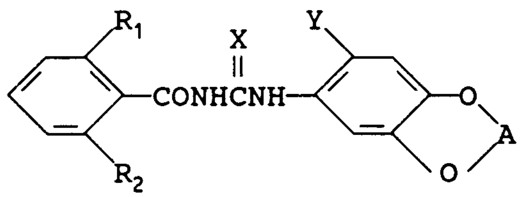

in which $R_1$ and $R_2$ may each represent a halogen atom, X may represent an oxygen atom, Y may represent a hydrogen atom and A represents an alkylene group substituted by a fluorine atom and optionally also by a chlorine atom.

The Applicants have now discovered a novel class of heterocyclic compounds which have both insecticidal and acaricidal activity.

In accordance with the present invention there is provided a compound of the general formula:

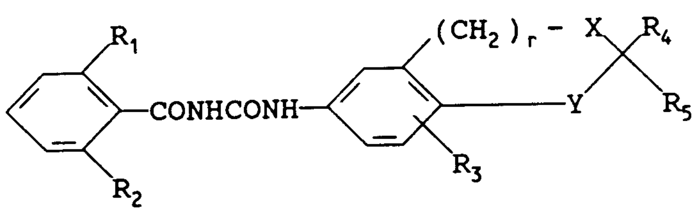

(I)

wherein $R_1$ represents a hydrogen or halogen atom; $R_2$ represents a halogen atom; $R_3$ represents a hydrogen or halogen atom or an alkyl group; $R_4$ and $R_5$ each independently represents a hydrogen atom or an alkyl, haloalkyl, alkoxy or aralkyl group; or $R_4$ and $R_5$ together form an alkylene group; X and Y each independently represents an oxygen or sulphur atom; and r is 0 or 1, with the proviso that at least one of $R_3$, $R_4$ and $R_5$ is not a hydrogen atom when both $R_1$ and $R_2$ represent a chlorine atom, X and Y both represent an oxygen atom and r is 0.

Preferably, any alkyl group or any alkyl component in any haloalkyl, alkoxy or aralkyl group has up to 6 carbon atoms.

It is particularly preferred that $R_1$ and $R_2$ each represents a fluorine atom; $R_3$ represents a hydrogen atom; $R_4$ represents a hydrogen atom, a $C_{1-4}$ alkyl group, a $C_{1-2}$ alkoxy group or a benzyl group; $R_5$ represents a hydrogen atom, or a $C_{1-5}$ alkyl or $C_{1-2}$haloalkyl group; or $R_4$ and $R_5$ together form a $C_{1-5}$ alkylene group.

In accordance with the present invention there is also provided a process for the preparation of a compound of general formula I which comprises hydrogenating a compound of the general formula:

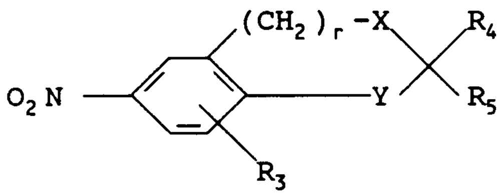

(VI)

to form a compound of the general formula:

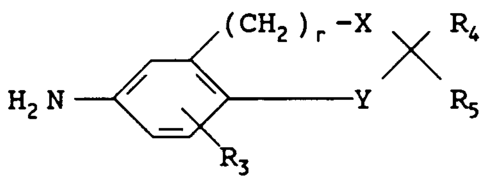

(II)

which is reacted with a compound of the general formula:

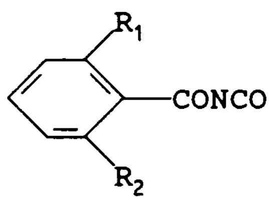

(III)

to form a compound of the general formula I, wherein $R_1$, $R_2$, $R_3$, $R_4$, $R_5$, X, Y and r have the meanings given above.

The hydrogenation of the compound of general formula VI is preferably carried out in the presence of a catalyst. Suitable catalysts are platinum or palladium catalysts, particularly a 5% platinum/charcoal catalyst.

The hydrogenation reaction may be carried out in the presence of a solvent or mixture of solvents. Suitable solvents are preferably ethers such as tetrahydrofuran, alcohols such as methanol and ethanol, and hydrocarbons such as toluene. Furthermore, a suitable base may be added as required. Suitable bases are alkali metal carbonates such as potassium carbonate.

The reaction is suitably carried out at a temperature in the range 0-150°C, preferably ambient temperature, and at a pressure of 60 psi or below.

The reaction between compounds of general formulae II and III is preferably carried out in the presence of a solvent. Suitable solvents are hydrocarbons such as benzene, toluene, xylene or petroleum fractions, chlorinated hydrocarbons such as chlorobenzene, chloroform, methylene chloride or dichloroethane, and ethers such as diethyl ether, dibutyl ether, dioxan and tetrahydrofuran. Mixtures of solvents are also suitable.

Preferably, the reaction is carried out at a temperature in the range 10-120°C.

Further purification of the product, where necessary, may be achieved by crystallisation from toluene or ethanol, or by chromatography on silica gel.

Some compounds of general formula VI are themselves novel and constitute a further aspect of the present invention. They may be prepared by reacting a compound of the general formula:

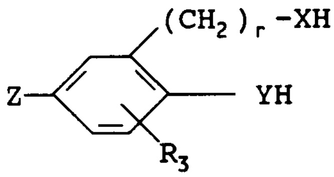

(IV)

with a compound of the general formula:

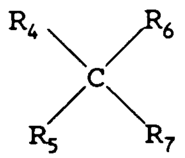

(V)

to form a compound of the general formula:

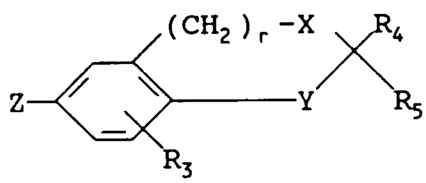

(VI)

wherein $R_3$, $R_4$, $R_5$, X, Y and r have the meanings given above; Z represents a hydrogen atom or a nitro group; and wherein (i) when r is 0 and $R_4$ represents an alkoxy group, $R_6$ and $R_7$ each represents the same moiety as $R_4$, or (ii) in all other cases, $R_6$ and $R_7$ together represent an oxygen atom, and in the case where Z is hydrogen, the compound of formula VI is further reacted with nitric acid to form the compound of formula VI in which Z is a nitro group.

The reaction between compounds of general formulae IV and V is preferably carried out in the presence of an acid catalyst, which may be any suitable inorganic or organic acid. Suitable acids include sulphonic acids, for example toluene sulphonic acid, carboxylic acids, for example benzoic acid, and mineral acids, for example hydrochloric acid.

The reaction is suitably carried out at a temperature in the range 40-120°C.

The reaction may be carried out in the presence of a solvent. Suitable solvents include any organic aprotic solvent such as tetrahydrofuran, hydrocarbons such as benzene and toluene, halogenated hydrocarbons, and ethers. Mixed solvents may also be used, such as, for example, toluene with dimethylsulphoxide.

Furthermore, the reaction is suitably carried out under nitrogen, especially where X and/or Y is a sulphur atom.

Compounds of general formula I exhibit pesticidal, particularly insecticidal and/or acaricidal, activity. Accordingly the invention also provides a pesticidal composition comprising a compound of general formula I in association with at least one inert carrier therefor. The invention further provides a method of combating pests at a locus, which comprises applying to the locus a pesticidal compound or composition according to the present invention.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is

subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry-flowable powders" consist of relatively small granules having a relatively high concentration of active ingredient. Emulsifiable concentrates usually contain, in addition to a solvent and, when necessary, cosolvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing pesticidal, herbicidal, or fungicidal properties. The compounds of the invention are especially useful when applied in admixture with other insecticides, especially organophosphates and pyrethroids. Mixtures with the commercial products fenvalerate, permethrin, cypermethrin, deltamethrin and alphamethrin are especially useful.

The following Examples illustrate the invention; Examples 1 to 40 illustrate the preparation of intermediates of general formula VI, while Examples 41 to 80 illustrate the preparation of compounds of general formula I.

## EXAMPLE 1

### Preparation of 2-propyl-6-nitro-1,3-benzodioxan

A stirred suspension of 2-hydroxy-5-nitrobenzyl alcohol (4.56g) in n-butyraldehyde (10 ml) containing a catalytic amount of benzoic acid (0.2g) was gradually warmed to reflux temperature under an atmosphere of dry nitrogen. The reaction mixture (which had become a clear solution) was maintained at this temperature for 4 hours. After cooling to ambient temperature, excess aldehyde was evaporated under reduced pressure. The residue was then dissolved in ethyl acetate (200 ml) and the solution washed successively

with saturated aqueous sodium bicarbonate and 25% aqueous sodium bisulphite before drying over anhydrous sodium sulphate. Evaporation of the dried solution under reduced pressure afforded an oily residue which crystallised on trituration with cold ethanol (30 ml). This material dissolved on warming and gave colourless crystals of pure product upon refrigeration (4.09g), mp 71-72°C. A second crop (1.0g), mp 70-72°C, of similar purity was obtained from the mother liquor (total yield 5.09g, 84%).

EXAMPLE 2

Preparation of 2-isopropyl-6-nitro-1,3-benzodioxan

An intimate mixture of 2-hydroxy-5-nitrobenzyl alcohol (3.0g), isobutyraldehyde (5.1g) and benzoic acid (0.1g) was stirred at reflux temperature under an atmosphere of nitrogen for 4 hours. Excess aldehyde was then removed by evaporation under reduced pressure and the residue was dissolved in ethyl acetate (200 ml). This solution was washed successively with 10% aqueous sodium carbonate, 25% aqueous sodium bisulphite and brine before drying over anhydrous sodium sulphate. Evaporation of the dried solution under reduced pressure afforded the crude product as an oil which solidified. Crystallisation from ethanol afforded the pure benzodioxan as a light brown crystalline solid (1.47g), mp 61-62°C. A second crop of crystals (1.0g) was obtained by concentrating the mother liquor (total yield 4.47g, 63%).

EXAMPLES 3 TO 8

By methods analogous to that of Example 1, further compounds of the general formula VI were prepared by reaction of compounds of general formula IV with compounds of general formula V. Details are given in Table I.

Table I

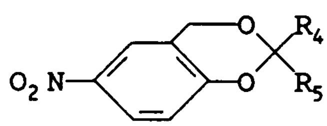

| Example No. | $R_4$ | $R_5$ | mp °C | Yield (%) |
|---|---|---|---|---|
| 3 | $C_2H_5$ | H | 57-58 | 63 |
| 4 | $nC_4H_9$ | H | 82-83 | 67 |
| 5 | $tC_4H_9$ | H | 82-83 | 23 |
| 6 | $CH_2C_6H_5$ | H | 118-119 | 79 |
| 7 | $-(CH_2)_5-$ | | 106-107 | 45 |
| 8 | $-(CH_2)_4-$ | | waxy solid | 23 |

EXAMPLE 9

Preparation of 2-propyl-6-nitro-1,3-benzoxathian

A solution of 2-hydroxy-5-nitrobenzyl mercaptan 2.0g) and n-butyraldehyde (1.56g) in toluene (80 ml) containing p-toluenesulphonic acid monohydrate (0.1g) was heated under reflux in a nitrogen atmosphere, separating the water formed in the reaction by means of a Dean-Stark apparatus. After 1 hour, the solvent

and excess aldehyde were removed by evaporation under reduced pressure to leave a dark oily residue which solidified upon trituration with cold ethanol. Recrystallisation of the crude solid from ethanol (decolourising with charcoal) afforded pure product as a light yellow-brown solid (1.6g, 62%), mp 99-100 °C.

EXAMPLES 10 TO 17

By methods analogous to that of Example 9, further compounds of the general formula VI were prepared by reaction of compounds of general formula IV with compounds of general formula V. Details are given in Table II.

Table II

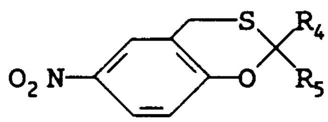

| Example No. | $R_4$ | $R_5$ | mp °C | Yield (%) |
|---|---|---|---|---|
| 10 | $C_2H_5$ | H | 80-81 | 39 |
| 11 | $iC_3H_7$ | H | oil | 54 |
| 12 | $nC_4H_9$ | H | 86-87 | 46 |
| 13 | $tC_4H_9$ | H | 81-82 | 66 |
| 14 | $-(CH_2)_5-$ | | 85-86 | 52 |
| 15 | $-(CH_2)_4-$ | | oil | 27 |
| 16 | $CH_3$ | $iC_3H_7$ | 52-54 | 38 |
| 17 | $CH_3$ | $CH_3$ | 102-103 | 7 |

EXAMPLE 18

Preparation of 2-isopropyl-6-nitro-3,1-benzoxathian

A solution of 2-mercapto-5-nitrobenzyl alcohol (2.79g) and isobutyraldehyde (5.43g) in benzene (80 ml) containing a catalytic amount of p-toluenesulphonic acid monohydrate (0.1g) was heated under reflux in a nitrogen atmosphere, separating the water formed in the reaction by means of a Dean-Stark apparatus. After 1.5 hours, the solvent and excess aldehyde were removed under reduced pressure and the resulting residue was purified by chromatography on silica gel using methylene chloride as eluant. The pure benzoxathian was obtained as white crystals (1.47g, 41%), mp 103-104 °C.

EXAMPLES 19 TO 24

By methods analogous to that of Example 18, further compounds of the general formula VI were prepared by reaction of compounds of general formula IV with compounds of general formula V. Details are given in Table III.

## Table III

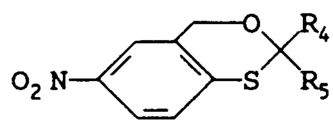

| Example No. | $R_4$ | $R_5$ | mp °C | Yield (%) |
|---|---|---|---|---|
| 19 | $tC_4H_9$ | H | 132-133 | 23 |
| 20 | $C_2H_5$ | H | 79-81 | 22 |
| 21 | $-(CH_2)_5-$ | | 121-123 | 18 |
| 22 | $-(CH_2)_4-$ | | 83-84 | 39 |
| 23 | $nC_3H_7$ | H | waxy solid | 33 |
| 24 | $CH_3$ | $CH_3$ | waxy solid | 49* |

* used without further purification - yield estimated by nmr

EXAMPLE 25

Preparation of 2-ethoxy-5-nitro-1,3-benzodioxole

4-Nitrocatechol (3.1g) was heated under reflux with triethyl orthoformate (15 ml) for 3.5 hours. The reaction mixture was then evaporated under reduced pressure and the resulting oily residue was purified by chromatography on silica gel using methylene chloride as eluant. Removal of the solvent from the appropriate fractions under high vacuum afforded the product as a light brown oil (3.5g, 83%).

EXAMPLE 26

By a method analogous to that of Example 25, a further compound of the general formula VI was prepared by reaction of a compound of general formula IV with a compound of general formula VII. Details are given in Table IV.

## Table IV

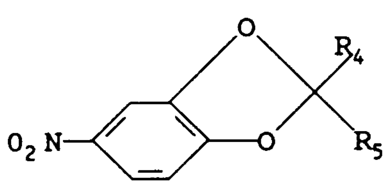

| Example No. | $R_4$ | $R_5$ | mp °C | Yield (%) |
|---|---|---|---|---|
| 26 | $OC_2H_5$ | $CH_3$ | oil | 90 |

EXAMPLE 27

Preparation of 2-tert-butyl-5-nitro-1,3-benzodioxole

(i) A solution of catechol (11.0g) and trimethylacetaldehyde (8.0g) in chloroform (100 ml) containing a catalytic amount of p-toluenesulphonic acid monohydrate (0.1g) was heated under reflux for 6 hours, separating the water formed in the reaction by means of a Dean-Stark trap. After removing the solvent under reduced pressure, the residue was purified by chromatography on silica gel using methylene chloride as eluant. The pure 2-tert-butyl-1,3-benzodioxole was isolated as volatile white crystals (8.5g, 51%), mp 40-42 ° C.
(ii) A solution of 2-tert-butyl-1,3-benzodioxole (3.6g) in 1,2-dichloroethane (20 ml) was added over 5 minutes to vigorously stirred 50% w/v nitric acid (25 ml) whilst maintaining the reaction temperature at 15-20 ° C by means of external cooling. After stirring at this temperature for 1 hour, the reaction mixture was poured into water (150 ml) and the resulting emulsion extracted with methylene chloride (2 x 100 ml). The combined extracts were washed with water and dried over anhydrous sodium sulphate. Evaporation of the dried extract afforded the crude nitro derivative which was purified by chromatography on silica gel using methylene chloride as eluant. The pure product was obtained as yellow crystals (3.7g, 83%), mp 31-32 ° C.

EXAMPLE 28

Preparation of 2,2-dimethyl-5-nitro-1,3-benzodioxole

(i) A solution of catechol (22.0g) in acetone (150 ml) containing a catalytic amount of p-toluenesulphonic acid monohydrate (0.2g) was heated under reflux for 2 days, passing the condensate through a Soxhlet apparatus containing 4A molecular sieves. Removal of the solvent under reduced pressure afforded a liquid residue which was distilled. Pure 2,2-dimethyl-1,3-benzodioxole was obtained as a colourless oil (13.7g, 46%), bp 81-83 ° C/15 mm.
(ii) A solution of 2,2-dimethyl-1,3-benzodioxole (10.0g) in 1,2-dichloroethane (15 ml) was added over 10 minutes to vigorously stirred 50% w/v nitric acid (25 ml) maintaining the temperature at 15-20 ° C by means of a cooling bath. After stirring at this temperature for 3/4 hour, the reaction mixture was poured into water (150 ml). The resulting emulsion was extracted with methylene chloride (3 x 100 ml) and the combined extracts washed with water (2 x 100 ml) and dried over anhydrous sodium sulphate. Evaporation of the dried extract afforded the crude benzodioxole which was purified by chromatographing on silica gel using methylene chloride as eluant. The pure product was thus obtained as pale yellow crystals (12.5g, 97%), mp 95-96 ° C.

EXAMPLE 29

Preparation of 2-methyl-2-(3-methylbut-1-yl)-5-nitro-1,3-benzodioxole

(i) A solution of catechol (22.0g) and 5-methyl-2-hexanone (34.2g) in toluene (150 ml) containing a catalytic amount of p-toluenesulphonic acid monohydrate (0.2g) was heated under reflux for 2 days, separating the water formed in the reaction by means of a Dean-Stark trap. The solvent was evaporated under reduced pressure and distillation of the residue afforded pure 2-methyl-2-(3-methylbut-1-yl)-1,3-benzodioxole as a colourless oil (40.1g, 97%), bp 116-117 ° C/15mm.
(ii) A solution of 2-methyl-2(3-methylbut-1-yl)1,3-benzodioxole (10.0g) in 1,2-dichloroethane (15 ml) was added over 10 minutes to vigorously stirred 50% w/v nitric acid, maintaining the temperature at 20-25 ° C by means of a cooling bath. The reaction was stirred at this temperature for 1 hour and then poured into

water (150 ml). The resulting emulsion was extracted with methylene chloride (3 x 100 ml) and the combined extracts were washed with water and dried over anhydrous sodium sulphate. Evaporation of the dried extract under reduced pressure afforded the crude product as an oil which was purified by distillation. The pure product was obtained as a pale yellow oil (23.7g, 94%), bp 130-133 °C/0.4mm.

EXAMPLES 30 TO 36

By methods analogous to that of Examples 27-29, further compounds of the general formula VI were prepared by reaction of compounds of general formula IV with compounds of general formula V. Details are given in Table V.

Table V

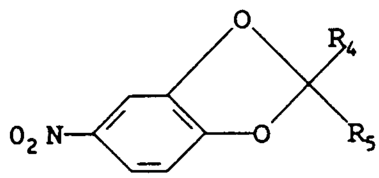

| Example No. | $R_4$ | $R_5$ | mp °C | Yield (%) |
|---|---|---|---|---|
| 30 | H | H | 154-156 | 71 |
| 31 | $CH_3$ | $iC_3H_7$ | oil | 93 |
| 32 | $-(CH_2)_4-$ | | 94-95 | 89 |
| 33 | $-(CH_2)_5-$ | | 107-109 | 37 |
| 34 | $iC_3H_7$ | $iC_3H_7$ | 54-56 | 97 |
| 35 | $C_2H_5$ | $iC_3H_7$ | oil | 87 |
| 36 | $CH_3$ | $CH(Cl)CH_3$ | oil | 76 |

EXAMPLE 37

Preparation of 5-nitrospiro[1,3-benzoxathiole-2,1'-cyclohexane]

A solution of 2-mercapto-4-nitrophenol (1.7g) and cyclohexanone (1.5g) in toluene (75 ml) containing a catalytic amount of p-toluenesulphonic acid monohydrate (0.1g) was heated under reflux for 6 hours, separating the water formed in the reaction by means of a Dean-Stark trap. Evaporation of the solvent followed by chromatography on silica gel, using methylene chloride as eluant, afforded the benzoxathiole as an orange oil (1.1g, 44%)*, which was used for the preparation of Example 77 without further purification.

EXAMPLES 38 TO 40

By methods analogous to that of Example 37, further compounds of the general formula VI were preapred by reaction of compounds of general formula IV with compounds of general formula V. Details are given in Table VI.

* yield based on nmr analysis

Table VI

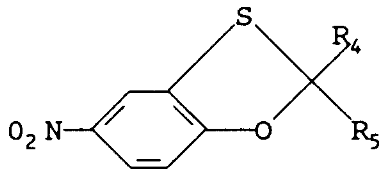

| Example No. | $R_4$ | $R_5$ | mp °C | Yield (%) |
|---|---|---|---|---|
| 38 | $CH_3$ | $iC_3H_7$ | oil | 39 * |
| 39 | $-(CH_2)_4-$ | | oil | 42 * |
| 40 | $CH_3$ | $CH_3$ | oil | 11 * |

* used without further purification - yield estimated by nmr

Elemental analysis data for the intermediates of general formula VI described above, where measured, is set out in Table VII below.

Table VII

| Example No. | Analysis (%) | | | | | |
|---|---|---|---|---|---|---|
| | C | | H | | N | |
| | Calc. | Found | Calc. | Found | Calc. | Found |
| 1 | 59.2 | 58.6 | 5.8 | 5.8 | 6.3 | 6.3 |
| 2 | 59.2 | 58.9 | 5.9 | 5.9 | 6.3 | 6.2 |
| 3 | 57.4 | 57.0 | 5.3 | 5.5 | 6.7 | 6.6 |
| 4 | 60.8 | 60.7 | 6.4 | 6.4 | 5.9 | 5.8 |
| 5 | 60.8 | 60.4 | 6.4 | 6.4 | 5.9 | 5.9 |
| 6 | 66.4 | 65.7 | 4.8 | 4.8 | 5.2 | 5.4 |
| 7 | 62.6 | 62.5 | 6.1 | 6.0 | 5.6 | 5.5 |
| 8 | 61.3 | 61.4 | 5.6 | 5.4 | 6.0 | 6.0 |
| 9 | 55.2 | 55.2 | 5.5 | 5.4 | 5.9 | 5.8 |
| 10 | 53.2 | 53.6 | 4.9 | 5.0 | 6.2 | 6.1 |

| | | | | | | |
|---|---|---|---|---|---|---|
| 11 | 55.2 | 55.8 | 5.5 | 5.6 | 5.9 | 5.6 |
| 12 | 56.9 | 56.7 | 6.0 | 6.1 | 5.5 | 5.5 |
| 13 | 56.9 | 57.3 | 6.0 | 6.0 | 5.5 | 5.5 |
| 14 | 58.9 | 58.8 | 5.7 | 5.5 | 5.3 | 5.3 |
| 16 | 56.9 | 57.0 | 6.0 | 6.0 | 5.5 | 5.5 |
| 17 | 53.5 | 53.5 | 4.9 | 4.8 | 6.2 | 6.2 |
| 18 | 55.2 | 55.1 | 5.5 | 5.6 | 5.9 | 5.7 |
| 19 | 56.9 | 56.8 | 6.0 | 5.9 | 5.5 | 5.5 |
| 20 | 53.3 | 53.6 | 4.9 | 5.0 | 6.2 | 6.1 |
| 21 | 58.9 | 58.8 | 5.7 | 5.6 | 5.3 | 5.3 |
| 22 | 57.4 | 57.0 | 5.2 | 5.1 | 5.6 | 5.5 |
| 23 | 55.2 | 55.7 | 5.5 | 5.4 | 5.9 | 6.0 |
| 25 | 51.2 | 51.2 | 4.4 | 4.3 | 6.8 | 6.6 |
| 26 | 53.3 | 53.4 | 4.9 | 5.0 | 6.2 | 6.4 |
| 27 | 59.2 | 58.9 | 5.8 | 5.8 | 6.3 | 6.1 |
| 28 | 55.4 | 55.9 | 4.6 | 4.5 | 7.2 | 7.0 |
| 29 | 62.2 | 62.4 | 6.8 | 6.8 | 5.6 | 5.6 |
| 30 | 50.3 | 50.3 | 3.0 | 3.1 | 8.4 | 8.2 |
| 31 | 59.2 | 59.8 | 5.8 | 6.0 | 6.3 | 6.3 |
| 32 | 59.7 | 59.9 | 5.0 | 5.1 | 6.3 | 6.0 |
| 33 | 61.3 | 61.7 | 5.5 | 5.6 | 6.0 | 6.1 |
| 34 | 62.1 | 62.3 | 6.8 | 6.5 | 5.6 | 5.7 |
| 35 | 60.6 | 60.2 | 6.3 | 6.3 | 5.9 | 6.0 |
| 36 | 49.3 | 49.1 | 4.1 | 4.1 | 5.7 | 5.6 |

EXAMPLE 41

Preparation of 2,6-difluoro-N-[[(2-propyl-1,3-benzodioxan-6-yl)amino]carbonyllbenzamide

A solution of 2-propyl-6-nitro-1,3-benzodioxan (2.23g) in ethanol (150 ml) was hydrogenated at ≦ 60 psi hydrogen pressure in the presence of 5% platinum/charcoal (0.2g) and anhydrous potassium carbonate (0.1g). The resulting reaction mixture was filtered and evaporated under reduced pressure. The residue was dried azeotropically by dissolving in toluene and evaporation under reduced pressure. The dry crude aniline thus obtained was redissolved in dry toluene (35 ml) and treated with 2,6-difluorobenzoyl isocyanate (2.0g), warming the reaction mixture briefly to reflux temperature. Upon cooling to 0-5 °C, the product crystallised out and was filtered off, washing briefly with cold toluene and light petroleum (bp 40-60 °C). Recrystallisation of this material from ethanol afforded the pure acylurea as colourless crystals (3.31g, 88%), mp 192-194 °C.

EXAMPLE 42

Preparation of 2,6-difluoro-N-[[(2-isopropyl-1,3-benzodioxan-6-yl]amino]carbonyl]benzamide

A solution of 2-isopropyl-6-nitro-1,3-benzodioxan (0.92g) in toluene (150 ml) was hydrogenated at ≦ 60

psi hydrogen pressure in the presence of 5% platinum/charcoal (0.2g) and anhydrous potassium carbonate (ca. 0.05g) at ambient temperature. The resulting reaction mixture was filtered and evaporated under reduced pressure to yield the crude aniline as a pale yellow oily residue. This material was redissolved in dry toluene (15 ml) and treated with 2,6-difluorobenzoyl isocyanate (0.75g). A precipitate of acylurea formed immediately and the suspension was stirred and briefly warmed to reflux temperature before allowing to cool to room temperature. After cooling to 0-5 °C, the precipitate of product was separated, washed with cold toluene followed by light petroleum and dried under vacuum at ≦ 60 °C. The pure acylurea was thus obtained as colourless crystals (1.36g, 88%), mp 201-202 °C.

EXAMPLES 43 TO 48

By methods analogous to that of Example 41, further compounds of the general formula I were prepared from intermediates of the general formula VI. Details are given in Table VIII.

Table VIII

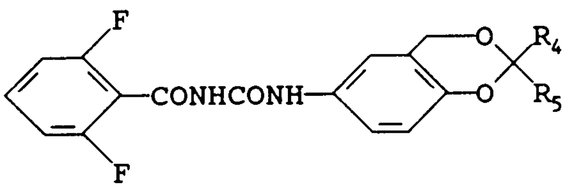

| Example No. | $R_4$ | $R_5$ | mp °C | Yield (%) |
|---|---|---|---|---|
| 43 | $C_2H_5$ | H | 184-185 | 80 |
| 44 | $nC_4H_9$ | H | 185-186 | 82 |
| 45 | $tC_4H_9$ | H | 228-229 | 91 |
| 46 | $CH_2C_6H_5$ | H | 177-178 | 66 |
| 47 | $-(CH_2)_5-$ | | 184-185 | 87 |
| 48 | $-(CH_2)_4-$ | | 173-175 | 72 |

EXAMPLE 49

Preparation of 2,6-difluoro-N-[[(2-propyl-1,3-benzoxathian-6-yl)amino]carbonyl]benzamide

A solution of 2-propyl-6-nitro-4H-1,3-benzoxathian (1.2g) in toluene (100 ml) was hydrogenated at ≦ 60 psi hydrogen pressure in the presence of 5% platinum/charcoal (0.2g) and anhydrous potassium carbonate (ca. 0.05g) at ambient temperature. The resulting reaction mixture was filtered and evaporated under reduced pressure to yield the crude aniline as a brown oil. This material was redissolved in dry toluene (40 ml) and treated with 2,6-difluorobenzoyl isocyanate (1.0g). A precipitate of acylurea formed immediately and reaction was completed by briefly warming to reflux temperature (which resulted in complete solution). Upon cooling to 0-5 °C, the pure product crystallised out and was separated, washed with cold toluene followed by light petroleum (bp 40-60 °C) and finally dried under vacuum at ≦ 60 °C. The pure acylurea was obtained as crystals (1.6g, 81%), mp 172-173 °C.

EXAMPLES 50 TO 57

By methods analogous to that of Example 49, further compounds of the general formula I were

prepared from intermediates of the general formula VI. Details are given in Table X.

Table X

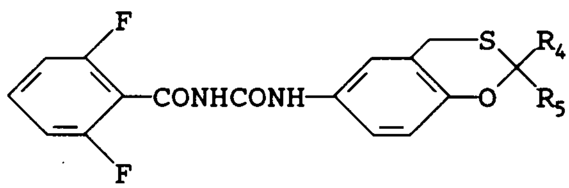

| Example No. | $R_4$ | $R_5$ | mp °C | Yield (%) |
|---|---|---|---|---|
| 50 | $C_2H_5$ | H | 201-210 | 79 |
| 51 | $iC_3H_7$ | H | 194-195 | 70 |
| 52 | $nC_4H_9$ | H | 200-201 | 83 |
| 53 | $tC_4H_9$ | H | 212-213 | 69 |
| 54 | $-(CH_2)_5-$ | | 189-190 | 57 |
| 55 | $-(CH_2)_4-$ | | 196-197 | 51 |
| 56 | $CH_3$ | $iC_3H_7$ | 185-186 | 75 |
| 57 | $CH_3$ | $CH_3$ | 177-178 | 76 |

EXAMPLE 58

Preparation of 2,6-difluoro-N-[[(2-isopropyl-3,1-benzoxathian-6-yl)amino]carbonyl]benzamide

A solution of 2-isopropyl-6-nitro-3,1-benzoxathian (1.25g) in toluene (200 ml) was hydrogenated at ≦ 60 psi hydrogen pressure in the presence of 5% platinum/charcoal (0.8g) and anhydrous potassium carbonate (ca. 0.01g). The resulting reaction mixture was filtered and evaporated under reduced pressure, removing the last traces of water from the residue by azeotropic evaporation with a fresh batch of toluene (50 ml). The crude amine was obtained as a yellow oil. This material was dissolved in dry toluene (20 ml) and 2,6-difluorobenzoyl isocyanate (1.05g) added with stirring. The reaction mixture was warmed briefly to reflux temperature and then cooled to 0-5 °C whereupon a precipitate of the acylurea was deposited. This was filtered off, washed briefly with cold toluene followed by petroleum ether (bp 40-60 °C) and dried under vacuum at 60 °C. The product was obtained as white crystals (1.35g, 66%), mp 210-212 °C (raised to 213-215 °C by recrystallisation from toluene).

EXAMPLES 59 TO 64

By methods analogous to that of Example 58, further compounds of the general formula I were prepared from intermediates of the general formula VI. Details are given in Table XI.

Table XI

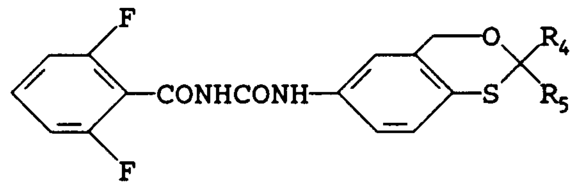

| Example No. | $R_4$ | $R_5$ | mp °C | Yield (%) |
|---|---|---|---|---|
| 59 | $tC_4H_9$ | H | 224-226 | 66 |
| 60 | $C_2H_5$ | H | 188-190 | 48 |
| 61 | $-(CH_2)_5-$ | | 182-183 | 59 |
| 62 | $-(CH_2)_4-$ | | 181-184 | 64 |
| 63 | $CH_3$ | $CH_3$ | 188-191 | 37 |
| 64 | $nC_3H_7$ | H | 176-178 | 24 |

EXAMPLE 65

Preparation of 2,6-difluoro-N-[[2-ethoxy-1,3-benzodioxol-5-yl)amino]carbonyl]benzamide

A solution of 2-ethoxy-5-nitro-1,3-benzodioxole (2.1g) in ethanol (100 ml) was hydrogenated at ≦60 psi hydrogen pressure in the presence of 5% platinum/charcoal (0.2g) and anhydrous potassium carbonate (0.2g) at ambient temperature. The resulting reaction mixture was filtered and the solvent was evaporated under reduced pressure. The residue was dried azeotropically by dissolving in toluene and evaporation under reduced pressure. The crude aniline thus obtained was redissolved in dry toluene (50 ml) and treated with 2,6-difluorobenzoyl isocyanate (2.0g). After stirring at reflux temperature for 1 hour, the reaction mixture was evaporated under reduced pressure to give a solid residue which was purified by chromatography on silica gel using methylene chloride/5% v/v methanol as eluant. The pure acylurea was obtained as colourless crystals (3.4g, 93%), mp 140-142 °C.

EXAMPLE 66

By a method analogous to that of Example 65, a further compound of the general formula I was prepared from an intermediate of the general formula VI.
Details are given in Table XII.

## Table XII

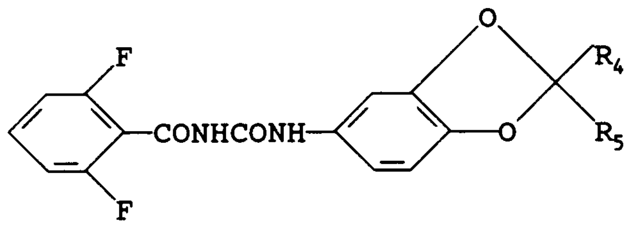

| Example No. | $R_4$ | $R_5$ | mp °C | Yield (%) |
|---|---|---|---|---|
| 66 | $OC_2H_5$ | $CH_3$ | 152-154 | 85 |

### EXAMPLE 67

Preparation of N-[[(2-tert-butyl-1,3-benzodioxol-5-yl)amino]carbonyl]-2,6-difluorobenzamide

A solution of 2-tert-butyl-5-nitro-1,3-benzodioxole (0.7g) in ethanol (100 ml) was hydrogenated at ≤ 60psi hydrogen pressure in the presence of 5% platinum/charcoal (0.2g) and anhydrous potassium carbonate (0.2g) at ambient temperature. The resulting reaction mixture was filtered through 'Hiflo supercel' (BDH) and evaporated under reduced pressure. The residue was dried azeotropically by dissolving in toluene and evaporating under reduced pressure. The crude aniline thus obtained was then redissolved in dry toluene (50 ml) and treated with 2,6-difluorobenzoyl isocyanate (0.63g). After stirring at reflux temperature for 1 hour, the reaction mixture was evaporated under reduced pressure. The resulting residue of crude acylurea was purified by chromatography on silica gel, using methylene chloride containing 2% (by volume) of methanol as eluant. The acylurea was obtained as a buff-coloured solid which, after trituration with ether, afforded the pure product as colourless crystals (0.7g, 62%), mp 176-178 °C.

### EXAMPLES 68 TO 76

By methods analogous to that of Example 67, further compounds of the general formula I were prepared from intermediates of the general formula VI. Details are given in Table XIII.

Table XIII

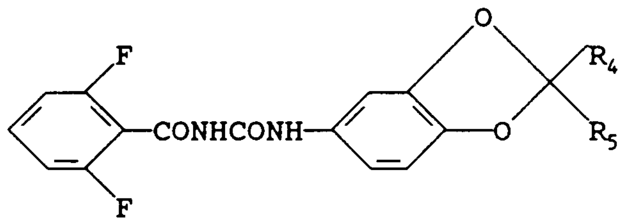

| Example No. | $R_4$ | $R_5$ | mp °C | Yield (%) |
|---|---|---|---|---|
| 68 | H | H | 199-200 | 97 |
| 69 | $CH_3$ | $CH_3$ | 201-203 | 95 |
| 70 | $CH_3$ | $iC_3H_7$ | 145-147 | 96 |
| 71 | $CH_3$ | $(CH_2)_2CH(CH_3)_2$ | 140-141 | 87 |
| 72 | $-(CH_2)_4-$ | | 222-223 | 88 |
| 73 | $-(CH_2)_5-$ | | 221-222 | 93 |
| 74 | $iC_3H_7$ | $iC_3H_7$ | 174-176 | 72 |
| 75 | $C_2H_5$ | $iC_3H_7$ | 156-158 | 97 |
| 76 | $CH_3$ | $CH(Cl)CH_3$ | 163-165 | 81 |

EXAMPLE 77

Preparation of 2,6-difluoro-N-[[spiro[1,3-benzoxathiole-2,1'-cyclohexan-5-yl]amino]carbonyl]benzamide

A solution of 5-nitrospiro[1,3-benzoxathiole-2,1'-cyclopexane] (0.8g) in toluene (150 ml) was hydrogenated at ≦60 psi hydrogen pressure in the presence of 5% platinum/charcoal catalyst (0.5g) at ambient temperature (2 days). The resulting reaction mixture was filtered through 'Hiflo supercel' (BDH) and 2,6-difluorobenzoyl isocyanate (0.65g) added to the filtrate. After stirring at ambient temperature for 1 hour, the solvent was removed under reduced pressure. The acylurea was isolated from the residue by chromatography on silica gel using methylene chloride/5% v/v methanol as eluant. Recrystallisation from diethyl ether/petroleum ether (bp 40-60 °C) afforded the pure product as white crystals (1.0g, 77%), mp 211-212 °C.

EXAMPLES 78 TO 80

By methods analogous to that of Example 77, further compounds of the general formula I were prepared from intermediates of the general formula VI. Details are given in Table XIV.

Table XIV

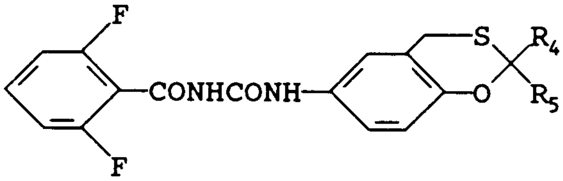

| Example No. | $R_4$ | $R_5$ | mp °C | Yield (%) |
|---|---|---|---|---|
| 78 | $CH_3$ | $iC_3H_7$ | 169-171 | 56 |
| 79 | $-(CH_2)_4-$ | | 180-181 | 46 |
| 80 | $CH_3$ | $CH_3$ | 200-202 | 24 |

Elemental analysis for the compounds of general formula I described above is set out in Table XV below

Table XV

| Example No. | Analysis (%) | | | | | |
|---|---|---|---|---|---|---|
| | C | | H | | N | |
| | Calc. | Found | Calc. | Found | Calc. | Found |
| 41 | 60.6 | 60.9 | 4.8 | 4.6 | 7.4 | 7.4 |
| 42 | 60.6 | 60.6 | 4.8 | 5.1 | 7.4 | 7.4 |
| 43 | 59.7 | 60.0 | 4.5 | 4.5 | 7.7 | 7.9 |
| 44 | 61.5 | 61.9 | 5.2 | 5.3 | 7.2 | 7.2 |
| 45 | 61.5 | 62.0 | 5.2 | 5.2 | 7.2 | 7.0 |
| 46 | 65.1 | 64.9 | 4.3 | 4.2 | 6.6 | 6.7 |
| 47 | 62.7 | 62.7 | 5.0 | 5.2 | 7.0 | 6.8 |
| 48 | 61.9 | 62.1 | 4.7 | 4.9 | 7.2 | 7.4 |
| 49 | 58.2 | 57.8 | 4.6 | 4.7 | 7.1 | 7.3 |
| 50 | 57.1 | 56.9 | 4.3 | 4.4 | 7.4 | 7.3 |
| 51 | 58.2 | 58.3 | 4.6 | 4.7 | 7.1 | 7.0 |
| 52 | 59.1 | 59.5 | 5.0 | 5.0 | 6.9 | 7.0 |
| 53 | 59.1 | 59.1 | 5.0 | 5.1 | 6.9 | 6.8 |
| 54 | 60.3 | 60.3 | 4.8 | 5.1 | 6.7 | 6.6 |
| 55 | 59.4 | 59.7 | 4.5 | 4.4 | 6.9 | 6.9 |
| 56 | 59.1 | 59.4 | 5.0 | 5.1 | 6.9 | 6.8 |
| 57 | 57.1 | 57.6 | 4.3 | 4.4 | 7.4 | 7.3 |
| 58 | 58.2 | 58.6 | 4.6 | 4.8 | 7.1 | 7.0 |
| 59 | 59.1 | 59.2 | 5.0 | 5.0 | 6.9 | 6.8 |
| 60 | 57.1 | 57.0 | 4.3 | 4.4 | 7.4 | 7.3 |
| 61 | 60.3 | 60.8 | 4.8 | 4.7 | 6.7 | 6.7 |
| 62 | 59.4 | 58.9 | 4.5 | 4.4 | 6.9 | 7.1 |
| 63 | 57.1 | 56.7 | 4.3 | 4.3 | 7.4 | 7.2 |
| 64 | 58.2 | 57.8 | 4.6 | 4.5 | 7.1 | 7.4 |
| 65 | 56.0 | 56.5 | 3.9 | 3.8 | 7.7 | 7.7 |
| 66 | 57.1 | 56.8 | 4.2 | 4.4 | 7.4 | 7.3 |
| 67 | 60.6 | 60.7 | 4.8 | 4.5 | 7.5 | 7.3 |
| 68 | 56.3 | 56.7 | 3.1 | 3.3 | 8.8 | 8.9 |
| 69 | 58.7 | 59.2 | 4.0 | 4.3 | 8.1 | 8.1 |

| | | | | | | |
|---|---|---|---|---|---|---|
| 70 | 60.6 | 61.1 | 4.8 | 4.9 | 7.5 | 7.5 |
| 71 | 62.4 | 62.4 | 5.5 | 5.4 | 6.9 | 7.1 |
| 72 | 61.0 | 61.4 | 4.3 | 4.4 | 7.5 | 7.4 |
| 73 | 61.9 | 61.8 | 4.6 | 4.6 | 7.2 | 7.2 |
| 74 | 62.4 | 62.2 | 5.5 | 5.5 | 6.9 | 7.0 |
| 75 | 61.5 | 61.8 | 5.1 | 5.0 | 7.2 | 7.2 |
| 76 | 54.8 | 55.0 | 3.8 | 3.7 | 7.1 | 6.9 |
| 77 | 59.4 | 59.7 | 4.5 | 4.5 | 6.9 | 7.0 |
| 78 | 58.2 | 58.4 | 4.6 | 4.6 | 7.1 | 7.1 |
| 79 | 58.4 | 58.0 | 4.1 | 3.9 | 7.1 | 6.9 |
| 80 | 56.0 | 55.5 | 3.9 | 3.9 | 7.7 | 7.7 |

## EXAMPLE 81

### Insecticidal Activity

Insecticidal activity of compounds of general formula I was assessed against the following pests:-

Spodoptera littoralis (Egyptian cotton leafworm)
Aedes aegypti (yellow fever mosquito)
Trialeurodes vaporariorum (greenhouse whitefly)

The test methods employed for each species appear below. In each test, unless otherwise stated, solutions or suspensions of test compound were made up over a range of concentrations in water (initially 0.1%w) containing 10%w acetone and 0.025%w "TRITON X-100" (trade mark) surface active agent (the condensation product of ethylene oxide with an alkyl phenol). These solutions were sprayed at a rate equivalent to 340 litres per hectare ($3.4 \times 10^{-5}$ $m^3/m^2$) onto Petri dishes containing either test species per se or diet onto which test species were subsequently introduced, as indicated. In some assays leaf discs infested with test species were sprayed whilst other assays involved the spraying of plants which were infested subsequently with test species after the spray solution had dried. The tests were all conducted under normal insectary conditions (23°C ± 2°C, fluctuating humidity and light).

The results of the testing at the initial test concentrations were graded, those compounds achieving 40-69% mortality being indicated by Grade B.

For compounds achieving 70-100% mortality, mortality assessments were made as indicated below, in terms of percentage mortality figures. In each test a $LC_{50}$ (the dosage of active material required to kill half of the test species) for the compound was calculated from the mortality figures and compared with the corresponding $LC_{50}$ for a standard insecticide, ethyl parathion, in the same test. The results are expressed as toxicity indices thus:

$$\text{toxicity index} = \frac{LC_{50}\ (\text{parathion})}{LC_{50}\ (\text{test compound})} \times 100$$

(i) Spodoptera littoralis (7 day) (Sl 7D)
Test solutions were sprayed as indicated above onto Petri dishes containing a nutritious diet for Egyptian cotton leafworm larvae. When the spray deposit had dried, each dish was infested with ten 2nd instar larvae. Mortality assessments were made 7 days after spraying.

(ii) Aedes aegypti (Aa)
Early 4th instar larvae were used. Test solutions were made up to 0.5 ppm of test compound (and progressive half-dilutions) in water containing 0.04%w "TRITON X-100" (trade mark); acetone was

initially present to aid solution, but was allowed to evaporate off before introduction of larvae. Ten early 4th instar larvae were placed in 100 ml of test solution held at 28°C, and after 48 hours, larval mortality was recorded. The final mortality was assessed by counting the number of emerged adult mosquitoes after one week.

(iii) Trialeurodes vaporariorum (Tv)

French bean plants (Phaseolus vulgaris) with two fully expanded leaves were placed in a breeding culture of T.vaporariorum, also on French bean plants, which were then disturbed to ensure resettlement on the introduced plants. During the subsequent 24 hour period, eggs were deposited and kept at 27°C, with 14 hours photoperiod. All adult whiteflies were then carefully removed, leaving egg samples of a known age. After eight days the majority of eggs had hatched. Leaf discs containing the newly hatched nymphs were then cut from the leaves and transferred to moist filter paper. The discs were examined under a low-powered microscope to determine the exact number of 1st instar nymphs per disc and to remove any unhatched eggs. On average, 70-100 nymphs were found per disc. The discs were transferred into Petri dishes and sprayed with test solutions as described above. After 6 days percentage mortalities were assessed.

**Table XVI**

**Insecticidal Activity**

| Compound of Example No. | Toxicity Index S.l. | A.a. | T.v. |
|---|---|---|---|
| 41 | 420 | 7 | |
| 42 | 40 | 41 | 1300 |

| | | | |
|---|---|---|---|
| 43 | 15 | 46 | |
| 44 | 64 | 26 | B |
| 45 | 120 | | |
| 46 | 65 | | B |
| 47 | | 16 | 3000 |
| 48 | 52 | 22 | 2900 |
| 49 | 120 | 24 | 3000 |
| 50 | | 20 | |
| 51 | 23 | | B |
| 52 | B | 7 | B |
| 53 | 80 | 2 | B |
| 54 | | 3 | B |
| 55 | B | 8 | |
| 56 | | 3 | 420 |
| 57 | | 7 | 430 |
| 58 | 16 | 11 | |
| 59 | 85 | | 990 |
| 60 | 28 | 16 | |
| 61 | | 6 | 2700 |
| 72 | | 7 | |
| 63 | B | | |
| 64 | | 38 | |
| 65 | B | | 89 |
| 66 | | | 44 |
| 67 | 150 | 37 | 1900 |
| 68 | 16 | 3 | |
| 69 | B | 36 | 2300 |
| 70 | 32 | 42 | 5900 |
| 71 | 480 | | 2100 |
| 72 | 21 | 6 | |
| 73 | | 57 | |
| 74 | B | 8 | B |
| 75 | | 32 | 2300 |
| 76 | 130 | 44 | 6900 |
| 77 | 30 | 64 | 6200 |

| | | | |
|---|---|---|---|
| 78 | 9 | | 8200 |
| 79 | 42 | 79 | 3800 |
| 80 | 33 | 37 | 130 |

EXAMPLE 82

Acaricidal Activity (mite life cycle)

Acaricidal activity of some compounds of formula I was assessed, employing adult female glasshouse red spider mites, Tetranychus urticae (T.u.), by the following procedure.

2cm diameter leaf discs cut from the leaves of French bean plants were placed, underside uppermost, on 5.5cm diameter filter papers, kept moist by a cotton wool wick dipped in water.

Each leaf disc was infested with 25 to 30 adult female mites which were removed after 6 hours, leaving about 50 eggs on each disc. Within 5 days the eggs hatched. The freshly emerged larvae on the leaf discs were sprayed with solutions of test compound made up as in Example 81 above, at a rate equivalent to 340 litres per hectare ($3.4 \times 10^{-5}$ $m^3/m^2$).

The discs were thereafter kept under normal laboratory conditions (23°C ± 2°C, fluctuating humidity and 16 hours day length). After 7 days assessment was made of the number of mites emerging as adults.

From the results the $LC_{50}$ was calculated, as given in Table XVII below.

Table XVII

| Acaricidal Activity | |
|---|---|
| Compound of Example No. | $LC_{50}$ (% active ingredient in spray) |
| 65 | 0.022 |

**Claims**

**1.** A compound of the general formula:

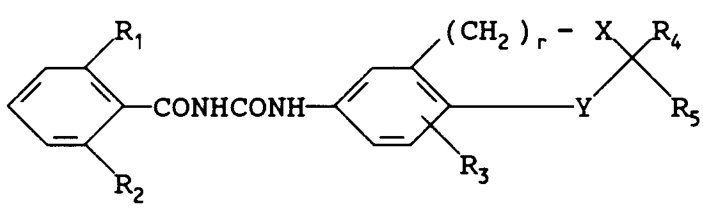

(I)

wherein $R_1$ represents a hydrogen or halogen atom; $R_2$ represents a halogen atom; $R_3$ represents a hydrogen or halogen atom or an alkyl group; $R_4$ and $R_5$ each independently represents a hydrogen atom or an alkyl, haloalkyl, alkoxy or aralkyl group; or $R_4$ and $R_5$ together form an alkylene group; X and Y each independently represents an oxygen or sulphur atom; and r is 0 or 1, with the proviso that at least one of $R_3$, $R_4$ and $R_5$ is not a hydrogen atom when both $R_1$ and $R_2$ represent a chlorine atom, X and Y both represent an oxygen atom and r is 0.

**2.** A compound as claimed in claim 1 wherein any alkyl group or any alkyl component in any haloalkyl, alkoxy or aralkyl group has up to 6 carbon atoms.

**3.** A compound as claimed in claim 2 wherein $R_1$ and $R_2$ each represents a fluorine atom; $R_3$ represents a

hydrogen atom; $R_4$ represents a hydrogen atom, a $C_{1-4}$ alkyl group, a $C_{1-2}$ alkoxy group or a benzyl group; $R_5$ represents a hydrogen atom, or a $C_{1-5}$ alkyl or $C_{1-2}$ haloalkyl group; or $R_4$ and $R_5$ together form a $C_{1-5}$ alkylene group.

**4.** A compound as claimed in claim 3 wherein $R_4$ represents a hydrogen atom or a methyl, ethyl, n-propyl, isopropyl, n-butyl, tert-butyl, ethoxy or benzyl group; $R_5$ represents a hydrogen atom or a methyl, isopropyl, methylbutyl or chloroethyl group; or $R_4$ and $R_5$ together form a $C_{4-5}$ alkylene group.

**5.** A process for the preparation of a compound as claimed in claim 1 which comprises hydrogenating a compound of the general formula:

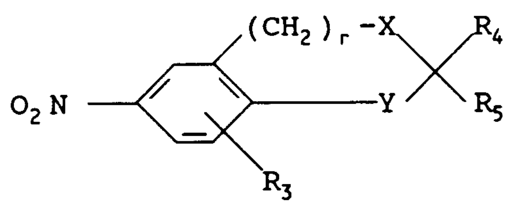

(VI)

to form a compound of the general formula:

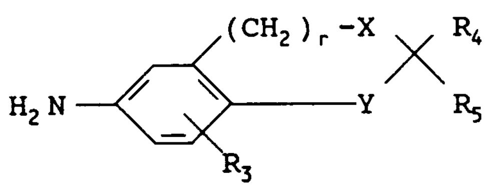

(II)

which is reacted with a compound of the general formula:

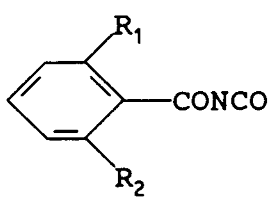

(III)

to form a compound of the general formula I, wherein $R_1$, $R_2$, $R_3$, $R_4$, $R_5$, X, Y and r have the meanings given in any one of claims 1 to 4.

**6.** A pesticidal composition comprising a compound as claimed in any one of claims 1 to 4, together with a carrier.

**7.** A composition as claimed in claim 6, which comprises at least two carriers, at least one of which is a surface-active agent.

**8.** A method of combating pests at a locus, which comprises applying to the locus a compound as claimed in any one of claims 1 to 4 or a composition as claimed in claim 6 or 7.

**9.** A compound of the general formula:

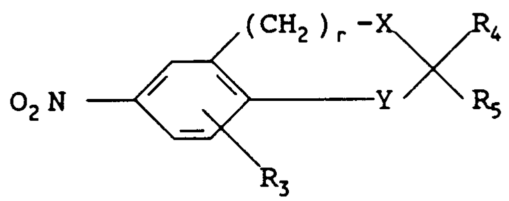

(VI)

wherein $R_3$, $R_4$, $R_5$, X, Y and r have the meanings given in claim 5.

**10.** A process for the preparation of a compound as claimed in claim 9 which comprises reacting a compound of the general formula:

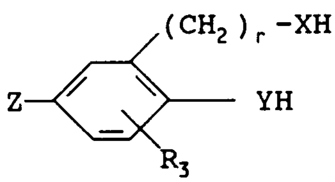

(IV)

with a compound of the general formula:

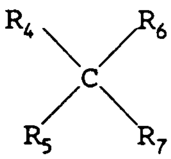

(V)

to form a compound of the general formula:

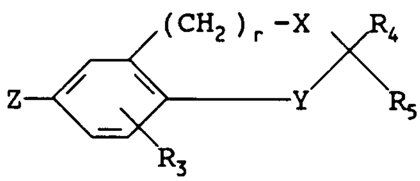

(VI)

wherein $R_3$, $R_4$, $R_5$, X, Y and r have the meanings given in claim 9; Z represents a hydrogen atom or a nitro group; and wherein (i) when r is 0 and $R_4$ represents an alkoxy group, $R_6$ and $R_7$ each represents the same moiety as $R_4$, or (ii) in all other cases, $R_6$ and $R_7$ together represent an oxygen atom, and in the case where Z is hydrogen, the compound of formula VI is further reacted with nitric acid to form the compound of formula VI in which Z is a nitro group.

European Patent Office

# EUROPEAN SEARCH REPORT

Application Number

EP 92 20 2225

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | GB-A-1 324 293 (PHILIPS)<br>* pages 1-3; page 10, lines 28-39 * | 1-6,8 | C07D317/46<br>C07D317/66<br>C07D317/72<br>C07D319/08<br>C07D327/04<br>C07D327/06<br>A01N43/30<br>A01N43/32<br>C07D317/62 |
| A | GB-A-1 531 407 (BAYER)<br>* page 1 * | 1,6,8 | |
| X | CHEMICAL ABSTRACTS, vol. 112, no. 7, 12 February 1990, Columbus, Ohio, US; abstract no. 52070y, T. ISOBE et al. 'Study on the antimicrobial activity of 1,3-dioxaindan derivatives (III). Antimicrobial activities of 5-nitro-2,2-dialkyl-1,3-dioxaindans' page 409 ;<br>& BOKIN BOBAI, 1989, 17(8), 365-369 | 9 | |
| X | JOURNAL OF HETEROCYCLIC CHEMISTRY, vol. 28, no. 3, April-May 1991, Provo, US, pages 625 - 634 I.M. TAKAKIS ET AL. 'Influence of the heterocyclic side ring on orientation during nitrations of 1,2-alkylenedioxy annelated benzenes and their mononitro derivatives'<br>* compound 4a * | 9,10 | **TECHNICAL FIELDS SEARCHED (Int. Cl.5)** |
| X | AUSTRALIAN JOURNAL OF CHEMISTRY, vol. 33, no. 3, March 1980, Melbourne, AU, pages 675 - 680 E.R. COLE ET AL. 'An improved method for the synthesis of 2,2-disubstituted and 2-monosubstituted 1,3-benzodioxoles'<br>* page 678 * | 9,10 | C07D |
| X | US-A-4 122 202 (F. FAURAN et al.)<br>* column 2 - column 3; examples 1-3,6,7 *<br>-/-- | 5,9,10 | |

The present search report has been drawn up for all claims

| Place of search | Date of completion of the search | Examiner |
|---|---|---|
| THE HAGUE | 28 SEPTEMBER 1992 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 92 20 2225
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CHIMIE THÉRAPEUTIQUE, vol. 7, no. 6, November-December 1972, Paris, FR, pages 434 - 443 M. ANTOINE ET AL. 'Acides m-dioxino quinoléine caboxyliques à action antibactérienne. I' * compounds 17,18, page 438, left column * | 5,9 | |
| X | JOURNAL OF THE AMERICAN CHEMICAL SOCIETY, vol. 88, no. 24, 20 December 1966, Washington, DC, US, pages 5855 - 5864 M.G. BURDON ET AL. 'Sulphoxide-carbodiimide reactions. IV. Acid-catalysed reactions of phenols with sulphoxides and carbodiimides' * compound 8 * | 9 | |
| X | GB-A-1 455 174 (BASF) * page 1; page 3, lines 15-16,19-20 * | 9,10 | TECHNICAL FIELDS SEARCHED (Int. Cl.5 ) |

The present search report has been drawn up for all claims

| Place of search | Date of completion of the search | Examiner |
|---|---|---|
| THE HAGUE | 29 SEPTEMBER 1992 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document